# EUROPÄISCHE PATENTSCHRIFT

(11) **EP 0 674 638 B1**
(45) Veröffentlichungstag und Bekanntmachung des Hinweises auf die Patenterteilung: **18.03.1998**
(21) Anmeldenummer: 94902722.1
(22) Anmeldetag: 08.12.1993
(51) Int. Cl.: C07D 471/04, A61K 31/435, A61K 31/34, A61K 31/38, A61K 31/40, A61K 31/47, A61K 31/045, A61K 31/415, A61K 31/22, A61K 31/35, A61K 31/66

(54) **SUBSTITUIERTE TRIOLE**
SUBSTITUTED TRIOLS
TRIOLS SUBSTITUES

(30) Priorität: 21.12.1992 DE 4243279
(43) Veröffentlichungstag der Anmeldung: 04.10.1995
(73) Patentinhaber: BAYER AG, 51368 Leverkusen (DE)
(72) Erfinder: ANGERBAUER, Rolf, D-42113 Wuppertal (DE); FEY, Peter, D-42111 Wuppertal (DE); HÜBSCH, Walter, D-42113 Wuppertal (DE); PHILIPPS, Thomas, D-51065 Köln (DE); BISCHOFF, Hilmar, D-421113 Wuppertal (DE); KRAUSE, Hans-Peter, D-58332 Schwelm (DE); PETERSEN VON GEHR, Jörg, D-44795 Bochum (DE); SCHMIDT, Delf, D-42113 Wuppertal (DE)
(86) Internationale Anmeldenummer: EP9303459
(87) Internationale Veröffentlichungsnummer: WO9414807

(56) Entgegenhaltungen:
- EP-A- 0 307 342
- EP-A- 0 326 386
- EP-A- 0 463 456
- EP-A- 0 465 970
- WO-A-84/02131
- US-A- 4 440 927
- US-A- 5 072 002
- JOURNAL OF MEDICINAL CHEMISTRY, vol. 36, Nr. 23, 1993, WASHINGTON, US; Seiten 3646 - 3657; C. CHAN ET AL.: 'Inhibitors of cholesterol biosynthesis. 1. 3,5-dihydroxy-7-(N-imidaz olyl)-6-heptenoates and -heptanoates, a novel series of HMG-CoA reductase inhibitors'

## Beschreibung

Die Erfindung betrifft substituierte Triole, Verfahren zu ihrer Herstellung, sowie ihre Verwendung in Arzneimitteln.

Es ist bekannt, daß aus Pilzkulturen isolierte Lacton-Derivate Inhibitoren der 3-Hydroxy-3-methyl-glutaryl Coenzym A Reduktase (HMG-CoA-Reduktase) sind [Mevinolin, EP 22 478; US-4 231 938].

Außerdem ist bekannt, daß Pyridin-substituierte Dihydroxyheptensäuren Inhibitoren der HMG-CoA-Reduktase sind [EP 325 130; EP 307 342; EP 306 929].

In EP-A-0 326 386 werden isochinolyn substituierte Dihydroxy-heptensäuren und deren Derivate offenbart. In U. S. 4,440,927 werden ebenfalls Dihydrpxy-heptensäuren und deren Derivate offenbart, die an Stelle des heterocyclischen Substituenten eine Biphenyleinheit tragen. Sowohl die Isochinolyn- als auch die biphenyl-substituierten Dihydroxy-heptensäure Derivate sind in vitro HMG-CoA-Reduktase Inhibitoren und eignen sich daher zur Behandlung von Hyperlipoproteenime.

Die vorliegende Erfindung betrifft substituierte Triole der allgemeinen Formel (I)

D-R (I)

in welcher
- D: für einen hetero- oder carbocyclischen Rest der Formel 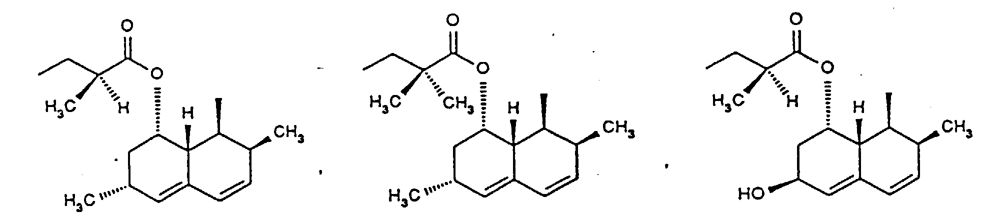 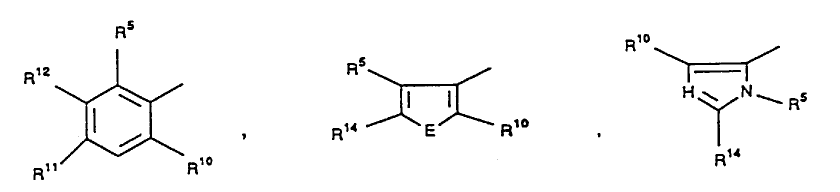 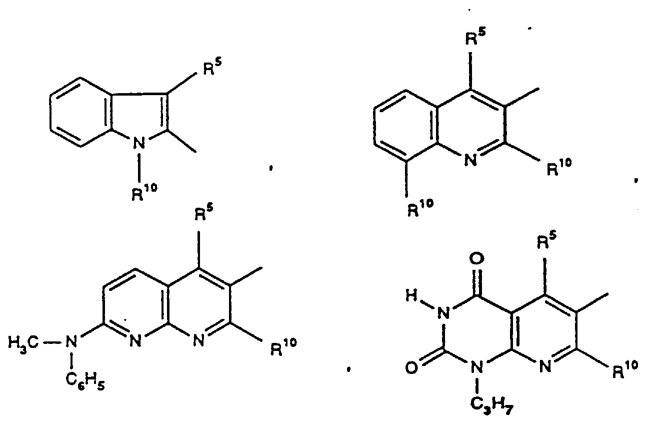 oder 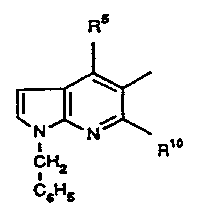 steht,
worin
- R⁵: Phenyl bedeutet, das gegebenenfalls durch Fluor oder Methyl substituiert ist,
- R¹⁰: Cyclopropyl oder geradkettiges oder verzweigtes Alkyl mit bis zu 3 Kohlenstoffatomen bedeutet,
- R¹¹: die oben genannte Bedeutung von R¹⁰ hat und mit diesem gleich oder verschieden ist oder Wasserstoff bedeutet,
- R¹²: Wasserstoff bedeutet,
- R¹⁴: Phenyl bedeutet
und
- E: ein Sauerstoff- oder Schwefelatom bedeutet oder eine Gruppe NR¹⁰ bedeutet,
und
- R: für einen Rest der Formel 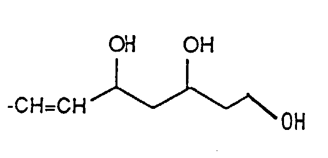
steht und deren Salze.

Im Rahmen der vorliegenden Erfindung werden physiologisch unbedenkliche Salze bevorzugt. Physiologisch unbedenkliche Salze der Chinolylmethoxyphenyl-essigsäureamide können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Die erfindungsgemäßen Verbindungen haben jeweils in Abhängigkeit der unter R aufgeführten Seitenketten, 1 oder 2 asymetrische Kohlenstoffatomen, an denen die Reste -OH gebunden sind. Sie können daher in verschiedenen stereochemischen Formen existieren.

Die Erfindung betrifft sowohl die einzelnen Isomeren als auch deren Mischungen. So können die erfindungsgemäßen Stoffe je nach der relativen Stellung der Reste -OR²¹ / -OR²² in der Erythro-Konfiguration oder in der Threo-Konfiguration vorliegen. Dies kann beispielhaft erläutert werden:

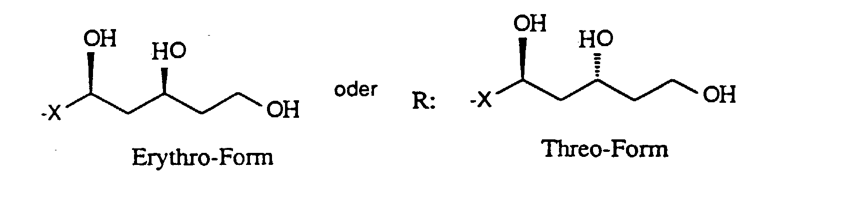

Sowohl von den Stoffen in Threo- als auch in Erythro-Konfiguration existieren wiederum jeweils zwei Enantiomere.

Darüber hinaus können die erfindungsgemäßen Stoffe aufgrund der Doppelbindung (X = -CH=CH-) in E-Konfiguration oder der Z-Konfiguration vorliegen. Bevorzugt sind diejenigen Verbindungen, die E-konfiguriert sind.

Außerdem wurde ein Verfahren zur Herstellung der erfindungsgemäßen Verbindungen der allgemeinen Formel (I) gefunden, dadurch gekennzeichnet, daß man

Verbindungen der allgemeinen Formel (II)

D-T (II)

in welcher
D die oben angegebene Bedeutung hat
und
- T: für einen Rest der Formel 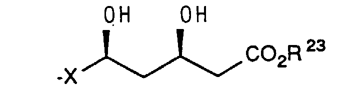 steht,
worin
- X: die Gruppe -CH=CH- bedeutet und R²³ eine Hydroxyschutzgruppe oder Wassestoff bedeutet,
in inerten Lösemitteln, unter Schutzgasatmosphäre, gegebenenfalls über die Stufe des Aldehyds, mit Reduktionsmitteln reduziert,

Das erfindungsgemäße Verfahren kann durch folgendes Formelschema beispielhaft erläutert werden:

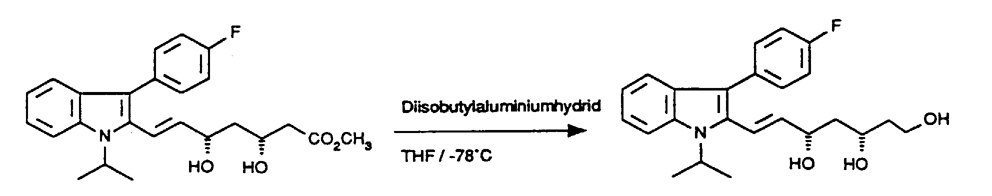

Als Lösemittel für die Reduktion eignen sich im allgemeinen organische Lösemittel. Bevorzugt sind Ether wie Diethylether, Tetrahydrofuran oder Dioxan. Bevorzugt ist Tetrahydrofuran.

Als Reduktionsmittel eignen sich Metallhydride, wie beispielsweise Lithiumaluminiumhydrid, Natriumcyanoborhydrid, Natriumaluminiumhydrid, Diisobutylaluminiumhydrid oder Natrium-bis-(2-methoxyethoxy)dihydroaluminat. Bevorzugt ist Düsobutylaluminiumhydrid.

Das Reduktionsmittel wird im allgemeinen in einer Menge von 4 mol bis 10 mol, bevorzugt von 4 mol bis 5 mol bezogen auf 1 mol der Verbindungen der allgemeinen Formel (II) eingesetzt.

Die Reduktion verläuft im allgemeinen in einem Temperaturbereich von -78°C bis +100°C, bevorzugt von -78°C bis 0°C, besonders bevorzugt bei -78°C, jeweils in Abhängigkeit von der Wahl des Reduktionsmittels.

Die Reduktion verläuft im allgemeinen bei Normaldruck, es ist aber auch möglich bei erhöhtem oder erniedrigtem Druck zu arbeiten.

Die Hydrierung erfolgt nach üblichen Methoden mit Wasserstoff in Anwesenheit von Edelmetallkatalysatoren, wie beispielsweise Pd/C, Pt/C oder Raney-Nickel in einem der oben aufgeführten Lösemittel, vorzugsweise in Alkoholen wie beispielsweise Methanol, Ethanol oder Propanol, in einem Temperaturbereich von -20°C bis +100°C, bevorzugt von 0°C bis +50°C, bei Normaldruck oder Überdruck.

Die Verbindungen der allgemeinen Formel (II) sind bekannt oder können nach üblichen Methoden hergestellt werden.

Im Fall der enantiomerenreinen Verbindungen der allgemeinen Formel (I) werden entweder die entsprechenden enantiomerenreinen Ester der allgemeinen Formel (II) eingesetzt, die nach publizierten Verfahren durch Umsetzung der racemischen Produkten mit enantiomerenreinen Aminen zu den entsprechenden diastereomeren Amidgemischen, anschließender Trennung durch Chromatographie oder Kristallisation in die einzelnen Diastereomere und anschließender Hydrolyse erhalten werden [vgl. DOS 40 40 026] oder indem man die racemischen Endprodukte nach üblichen chromatographischen Methoden trennt.

Die erfindungsgemäßen substituierten Hetero- und Carbocyclischen Triole sowie deren isomere Formen besitzen wertvolle, im Vergleich zum Stand der Technik überlegene, pharmakologische Eigenschaften, insbesondere sind sie hochwirksame Inhibitoren der 3-Hydroxy-3-methyl-glutaryl Coenzym A (HGM-CoA) Reduktase und infolge dessen Inhibitoren der Cholesterolbiosynthese. Sie können deshalb zur Behandlung von Hyperlipoproteinämie oder Artheriosklerose eingesetzt werden. Die erfindungsgemäßen Wirkstoffe bewirken außerdem eine Senkung des Cholesteringehaltes im Blut.

Die pharmakologische Wirkung der erfindungsgemäßen Stoffe wurden in folgendem Test bestimmt:

### Biologischer Test für HMGCoA-Reduktaseinhibitoren

Cholesterin wird im Säugetierorganismus aus Acetateinheiten aufgebaut. Um die hepatische Cholesterinbiosynthese in vivo zu messen, wurde den Tieren radioaktiv markiertes ¹⁴C-Acetat appliziert und später der Gehalt an ¹⁴C-Cholesterin in der Leber bestimmt.

Die zu untersuchenden Substanzen wurden auf eine Hemmung der hepatischen Cholesterinbiosynthese in vivo an männlichen Wistar-Ratten mit einem Körpergewicht zwischen 140 und 160 g geprüfL Die Ratten wurden zu diesem Zweck 18 Stunden vor der oralen Applikation der Substanzen gewogen, in Gruppen zu 6 Tieren (Kontrollgruppe ohne Substanzbelastung 8 Tiere) eingeteilt und nüchtemgesetzt. Die zu untersuchenden Substanzen wurden direkt vor der Applikation in wäßriger 0,75 %iger Traganthsuspension mit einem Ultra-Turrax suspendiert. Die Applikation der Traganthsuspension (Kontrolltiere) bzw. der in Traganth suspendierten Substanzen erfolgte mittels einer Schlundsonde. 2 Stunden der oralen Substanzgabe wurde den Tieren ¹⁴C-Acetat (12,5 µCi/Tier) intraperitoneal injiziert.

Weitere 2 Stunden später (4 Stunden nach Substanzapplikation wurden die Tiere durch Halsschnitt getötet und entblutet. Anschließend wurde der Bauchraum geöffnet und eine Leberprobe von ca 700 mg zur Bestimmung des aus ¹⁴C-Cholesterins entnommen. Die Extraktion des Cholesterins erfolgte modifiziert nach Duncan et al. (J. Chromatogr. 162 (1979) 281-292). Die Leberprobe wurde in einem Glaspotter in Isopropanol homogenisiert. Nach Schütteln und anschließender Zentrifugation wurde der Überstand mit alkoholischer KOH versetzt und die Cholesterinester verseift. Nach der Verseifung wurde das Gesamtcholesterin mit Heptan ausgeschüttelt und der Überstand eingedampft Der Rückstand wurde in Isopropanol aufgenommen, in Szintillationsröhrchen überführt und mit LSC-Cocktail aufgefüllt. Das aus ¹⁴C-Acetat in der Leber synthetisierte ¹⁴C-Cholesterin wurde im Flüssigkeitsszintillationszähler gemessen. Der hepatische ¹⁴C-Cholesteringehalt, der nur mit Traganth behandelten Tiere diente als Kontrolle. Die inhibitorische Aktivität der Substanzen wird in % des synthetisierten hepatischen ¹⁴C-Cholesteringehaltes der Traganth-Kontrolltiere (=100 %) angegeben.

Zur vorliegenden Erfindung gehören auch pharmazeutische Zubereitungen, die neben inerten, nicht-toxischen, pharmazeutisch geeigneten Hilfs- und Trägerstoffen eine oder mehrere Verbindungen der allgemeinen Formel (I) enthalten, oder die aus einem oder mehreren Wirkstoffen der Formel (I) bestehen, sowie Verfahren zur Herstellung dieser Zubereitungen.

Die Wirkstoffe der Formel (I) sollen in diesen Zubereitungen in einer Konzentration von 0,1 bis 99,5 Gew.-%, bevorzugt von 0,5 bis 95 Gew.-% der Gesamtmischung vorhanden sein.

Neben den Wirkstoffen der Formel (I) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs- oder Trägerstoffen.

Im allgemeinen hat es sich als vorteilhaft erwiesen, den oder die Wirkstoffe der Formel (I) in Gesamtmengen von etwa 0,1 µg/kg bis etwa 100 µg/kg, bevorzugt in Gesamtmengen von etwa 1 µg/kg bis 50 µg/kg Körpergewicht je 24 Stunden, gegebenenfalls in Form mehrerer Einzelgaben, zur Erzielung des gewünschten Ergebnisses zu verabreichen.

Es kann aber gegebenenfalls vorteilhaft sein, von den genannten Mengen abzuweichen, und zwar in Abhängigkeit von der Art und dem Körpergewicht des behandelten Objekts, vom individuellen Verhalten gegenüber dem Medikament, der Art und Schwere der Erkrankung, der Art der Zubereitung und Applikation, sowie dem Zeitpunkt bzw. Intervall, zu welchem die Verabreichung erfolgt.

### Herstellungsbeispiele

### Beispiel 1

Erythro(E)-7-[1-Benzyl-4(4-fluorphenyl)-6-isopropyl-1H-pyrrolo[2,3-b]pyridin-5-yl]hept-6-en-1,3,5-triol

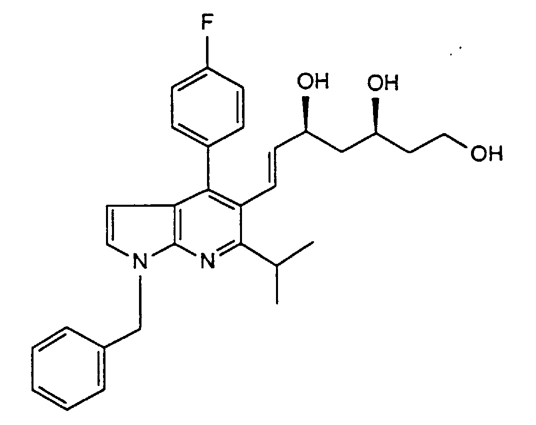

Zu einer Lösung von 207 mg (0,4 mmol) Methyl-erythro(E)-7-[4-(4-fluorphenyl-6-isopropyl-1H-pyrrolo[2,3-b]pyridin-5-yl]-3,5-dihydroxy-hept-6-enoat in 5 ml wasserfreiem THF tropft man unter Argon bei -78°C 1,33 ml (2 mmol) einer 1,5 M Düsobutyl-aluminiumhydrid-Lösung in Toluol. Man rührt 2,5 h bei -78°C, läßt 16 h bei -30°C stehen, versetzt dann nach Entfemen der Kühlung vorsichtig mit 5 ml Waser und 2 ml 1 N Salzsäure und extrahiert mit Essigester. Der kolloidale Niederschlag wird über Kieselgur abgesaugt die organische Phase mit ges. Kochsalzlösung gewaschen, über Natriumsulfat getrocknet und eingeengt. Nach Chromatographie in 15 g Kieselgel (230-400 mesh) mit Methylenchlorid-Essigester (1:2) erhält man 47 mg (38 %) eines farbloses Öls.
FAB-MS: 489 (100 %, M+H)

### Analog wurden hergestellt:

### Beispiel 2

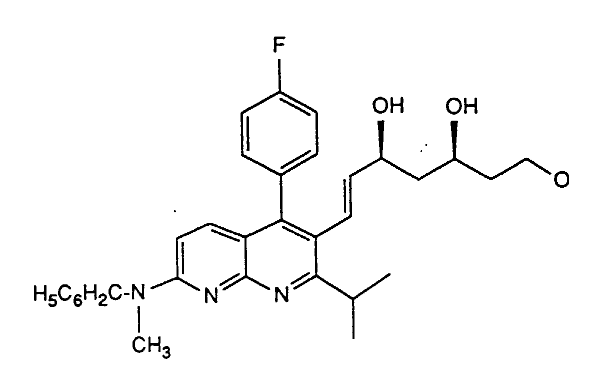

Ausbeute: 43 %
amorph
MS-Daten: 530 (79 %, M + 1) FAB

### Beispiel 3

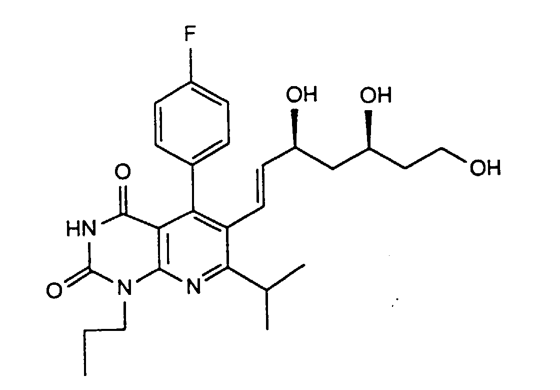

Ausbeute: 31 %
amorph
MS-Daten: 486 (100 %, M + 1) DCI

### Beispiel 4

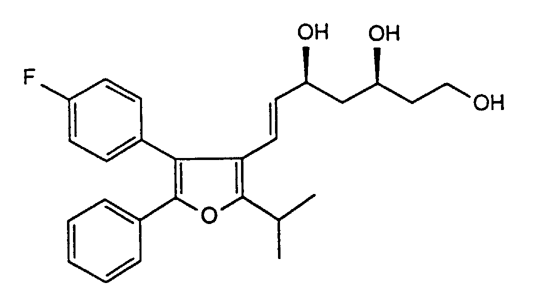

Ausbeute: 24 %
Fp.: 119°C
MS-Daten: 424 β 5 %, M + ) DCI

### Beispiel 5

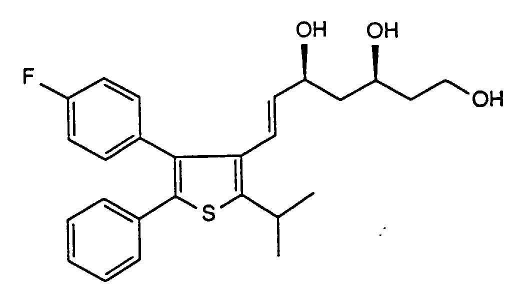

Ausbeute: 30 %
Fp.: 114°C
MS-Daten: 440 (25 %, M +) DCI

### Beispiel 6

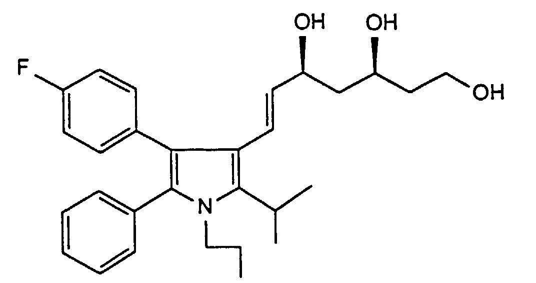

Ausbeute: 26 %
amorph
MS-Daten: 465 (60 %, M +) FAB

### Beispiel 7

Erythro-(E)-7-[2-cyclopropyl-4-(4-fluorphenyl)-8-methyl-chinolin-3-yl]-hept-6-ene-1,3,5-triol 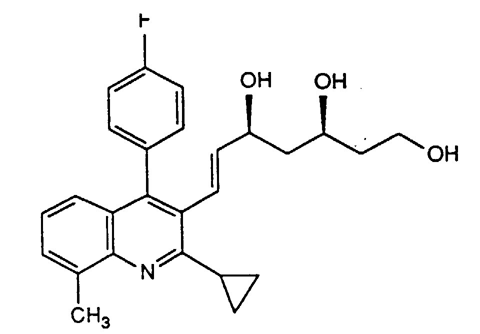 225 mg (0,5 mmol) Methyl-erythro-(E)-7-[2-cyclopropyl-4-(4-fluorphenyl)-8-methyl-chinolin-3-yl]-3,5-dihydroxy-hept-6-enoat werden in 20 ml absol. Tetrahydrofuran gelöst und nach Zugabe von 76 mg (2 mmol) Natriumborhydrid wird 4 h unter Rückfluß gekocht. Nach Abkühlen der Reaktionslösung gibt man 10 ml gesättigte Ammoniumchloridlösung und 10 ml H₂O zu und extrahiert dann 3 mal mit je 50 ml Essigester. Die organische Phase wird mit gesättigter NaCl-Lösung gewaschen, mit Na₂SO₄ getrocknet und am Rotationsverdampfer eingeengt. Nach Chromatographie über Kieselgel 60 (25-40 µ, Laufmittel: Essigester/Petrolether 8/2) erhält man das gewünschte Produkt
Ausbeute: 73 mg (35 % der Theorie), Fp. 148°C
FAB-MS: 422 (100%,M + H)

### Beispiel 8

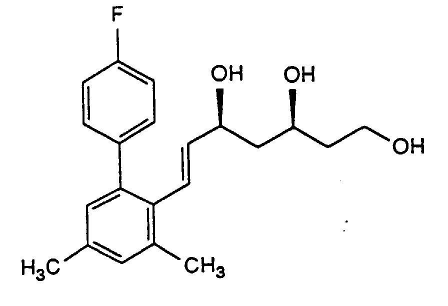

Ausbeute: 63 %
amorph
MS-Daten: (25 %, M +) FAB

### Beispiel 9

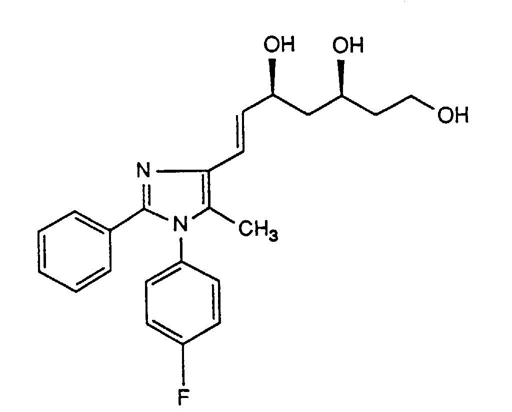

Ausbeute: 71 %
amorph
MS-Daten: (100 %, M + 1) FAB

### Beispiel 10

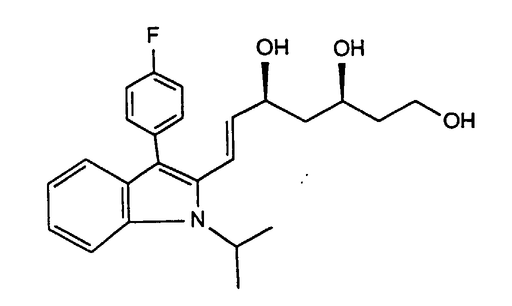

Ausbeute: 60 %
amorph
MS-Daten (100 %, M +) FAB

## Patentansprüche

1. Substituierte Triole der allgemeinen Formel I
D-R
in welcher
D für einen hetero- oder carbocyclishen Rest der Formel 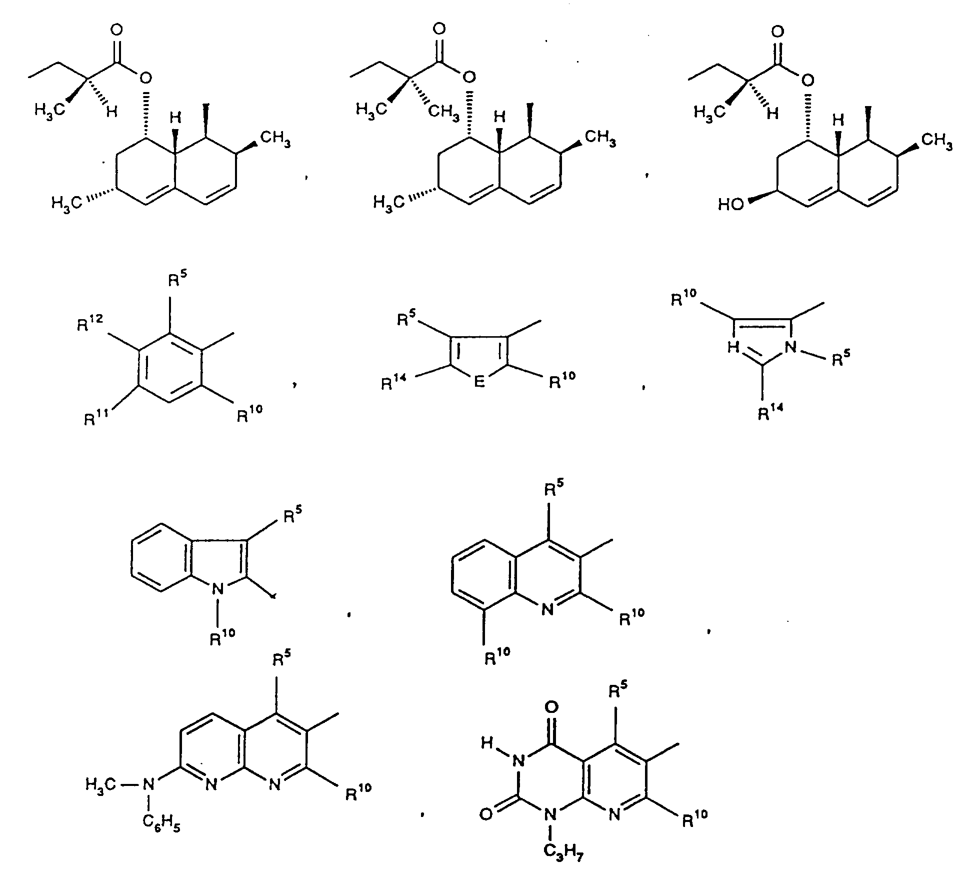
oder 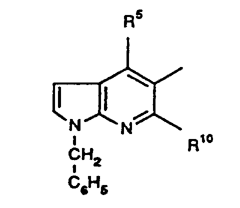 steht, worin
R⁵ Phenyl bedeutet, das gegebenenfalls durch Fluor oder Methyl substituiert ist,
R¹⁰ Cyclopropyl oder geradkettiges oder verzweigtes Alkyl mit bis zu 3 Kohlenstoffatomen bedeutet,
R¹¹ die oben genannte Bedeutung von R¹⁰ hat und mit diesem gleich oder verschieden ist oder Wasserstoff bedeutet,
R¹² Wasserstoff bedeutet,
R¹⁴ Phenyl bedeutet
und
E ein Sauerstoff- oder Schwefelatom bedeutet oder eine Gruppe NR¹⁰ bedeutet,
und
R für einen Rest der Formel 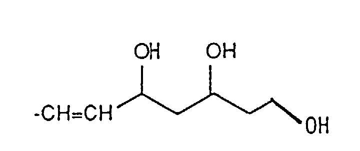
steht und deren Salze.

2. Substituierte Triole nach Anspruch 1 zur therapeutischen Anwendung.

3. Verfahren zur Herstellung von substituierten Triolen nach Anspruch 1 dadurch gekennzeichnet, daß man
Verbindungen der allgemeinen Formel II
D-T
in welcher
D die oben angegebene Bedeutung hat
und
T für einen Rest der Formel 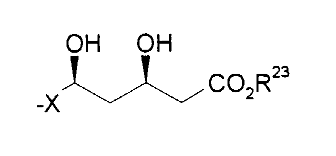 steht,
worin
X die Gruppe -CH=CH- bedeutet und
R²³ eine Hydroxyschutzgruppe oder Wasserstoff bedeutet,
in inerten Lösemitteln, unter Schutzgasatmospäre, gegebenenfalls über die Stufe des Aldehyds, mit Reduktionsmitteln reduziert.

4. Arzneimittel enthaltend mindestens ein substituiertes Triol nach Anspruch 1.

5. Arzneimittel nach Anspruch 4 zur Behandlung von Hyperlipoproteinämie.

6. Verfahren zur Herstellung von Arzneimitteln nach Anspruch 4 und 5 dadurch gekennzeichnet, daß man die substituierten Triole gegebenenfalls mit Hilfe üblicher Hilfs- und Trägerstoffe in eine geeignete Applikationsform überführt.

7. Verwendung von substituierten Triolen nach Anspruch 1 zur Herstellung von Arzneimittel.

8. Verwendung nach Anspruch 7 zur Herstellung von Arzneimitteln zur Behandlung von Hyperlipoproteinämie.

## Claims

1. Substituted triols of the general formula I
D-R
in which
D represents a hetero- or carbocyclic radical of the formula 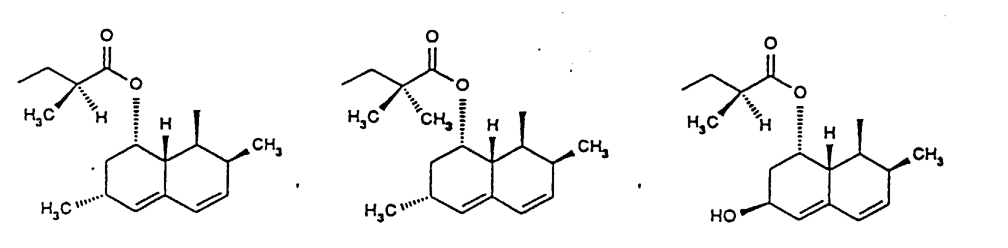 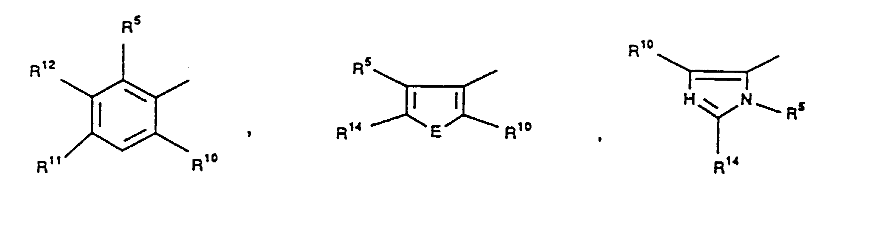 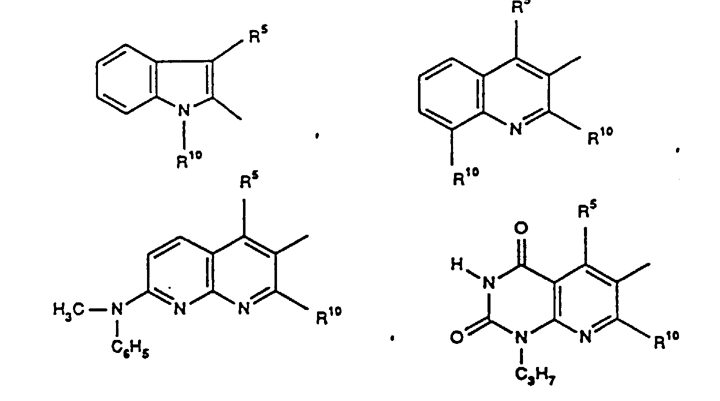 or 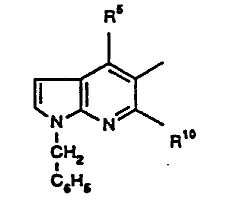
wherein
R⁵ denotes phenyl which is optionally substituted by fluorine or methyl,
R¹⁰ denotes cyclopropyl or straight-chain or branched alkyl having up to 3 carbon atoms,
R¹¹ has the abovementioned meaning of R¹⁰ and is identical to or different from this or denotes hydrogen,
R¹² denotes hydrogen,
R¹⁴ denotes phenyl
and
E denotes an oxygen or sulphur atom, or denotes a group NR¹⁰,
and
R represents a radical of the formula 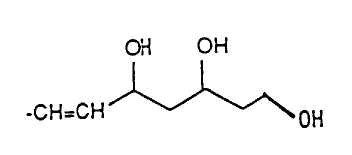 and their salts.

2. Substituted triols according to Claim 1 for therapeutic use.

3. Process for the preparation of substituted triols according to Claim 1, characterized in that
compounds of the general formula II
D-T
in which
D has the abovementioned meaning
and
T represents a radical of the formula 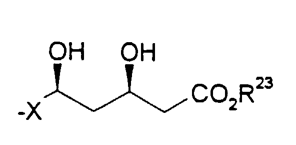 wherein
X denotes the group -CH = CH- and
R²³ denotes a hydroxy protective group or hydrogen,
are reduced with reducing agents in inert solvents, under a protective gas atmosphere, optionally via the stage of the aldehyde.

4. Medicaments containing at least one substituted triol according to Claim 1.

5. Medicaments according to Claim 4 for the treatment of hyperlipoproteinaemia.

6. Process for the production of medicaments according to Claim 4 and 5, characterized in that the substituted triols are converted into a suitable administration form, if appropriate with the aid of customary auxiliaries and excipients.

7. Use of substituted triols according to Claim 1 for the production of medicaments.

8. Use according to Claim 7 for the production of medicaments for the treatment of hyperlipoproteinaemia.

## Revendications

1. Triols substitués de formule générale I
D-R
dans laquelle
D représente un reste hétérocyclique ou carbocyclique de formule 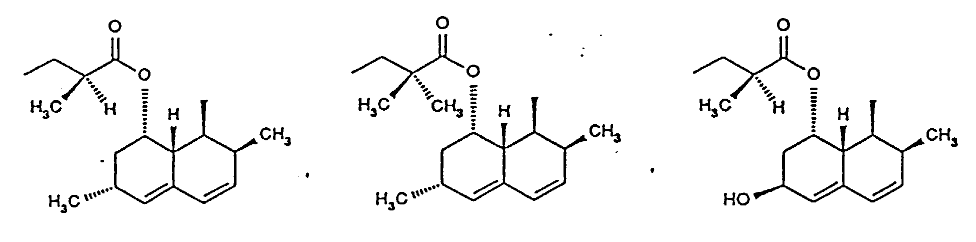 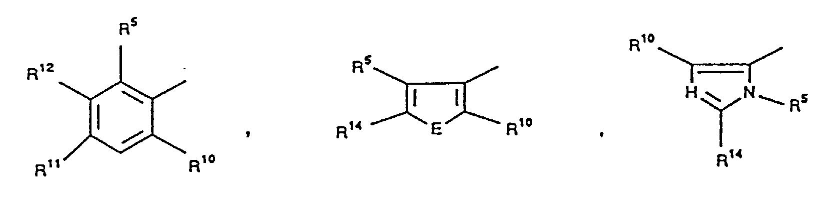 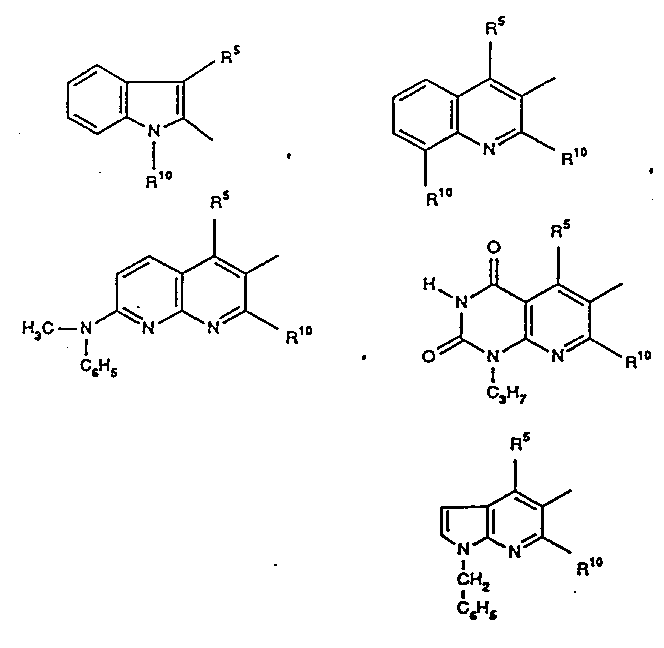
dans laquelle
R⁵ désigne un groupe phényle qui est éventuellement substitué par du fluor ou par un radical méthyle,
R¹⁰ est un groupe cyclopropyle ou un grupe alkyle linéaire ou ramifié ayant jusqu'à 3 atomes de carbone,
R¹¹ a la définition indiquée ci-dessus pour R¹⁰ et est identique à ce dernier ou en est différent ou représente de l'hydrogène,
R¹² représente de l'hydrogène,
R¹⁴ est un groupe phényle,
et
E désigne un atome d'oxygène ou de soufre ou un groupe NR¹⁰
et
R représente un reste de formule 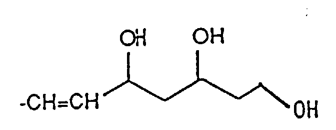 et leurs sels.

2. Triols substitués suivant la revendication 1, destinés à une application thérapeutique.

3. Procédé de production de triols substitués suivant la revendication 1, caractérisé en ce qu'on réduit avec des agents réducteurs des composés de formule générale II
D-T
dans laquelle
D a la définition indiquée ci-dessus
et
T représente un reste de formule 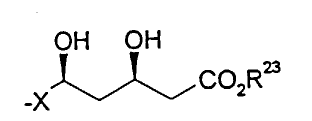
où
X représente le groupe -CH=CH- et
R²³ désigne un groupe protégeant la fonction hydroxy ou représente de l'hydrogène,
dans des solvants inertes, sous atmosphère de gaz protecteur, en passant éventuellement par le stade de l'aldéhyde.

4. Médicament contenant au moins un triol substitué suivant la revendication 1.

5. Médicament suivant la revendication 4, destiné au traitement de l'hyperlipoprotéinémie.

6. Procédé de préparation de médicaments suivant les revendications 4 et 5, caractérisé en ce qu'on fait prendre aux triols substitués une forme administrable approppriée, le cas échéant, à l'aide de substances auxiliaires et de supports classiques.

7. Utilisation de triols substitués suivant la revendication 1 pour la préparation de médicaments.

8. Utilisation suivant la revendication 7 pour la préparation de médicaments destinés au traitement de l'hyperlipoprotéinémie.
